# EUROPEAN PATENT SPECIFICATION

(11) **EP 3 024 514 B1**
(45) Date of publication and mention of the grant of the patent: **14.06.2017**
(21) Application number: 14777783.3
(22) Date of filing: 21.07.2014
(51) Int. Cl.: A61M 5/158, A61M 5/32, A61M 25/06

(54) **BUTTERFLY NEEDLE WITH A PROTECTIVE DEVICE**
SCHMETTERLINGSNADEL MIT EINER SCHUTZVORRICHTUNG
AIGUILLE À AILETTES ÉQUIPÉE D'UN DISPOSITIF DE PROTECTION

(30) Priority: 22.07.2013 IT MO20130211
(43) Date of publication of application: 01.06.2016
(73) Proprietor: Delta Med S.P.A. Unipersonale, 46019 Viadana (Mantova) (IT)
(72) Inventor: BERTOLI, Alessandro, 26037 San Giovanni In Croce (CR) (IT); BALBONI, Alessandro, 46030 San Giorgio di Mantova (MN) (IT)
(74) Representative: Bergamini, Silvio
(86) International application number: PCT/IB2014/063273
(87) International publication number: WO 2015/011628

(56) References cited:
- EP-A1- 2 204 204
- US-B1- 6 749 588

## Description

### Field of the Invention

The invention relates to a butterfly needle with a protective device, generally for use in the healthcare industry to prevent operators from being injured during handling after use.

### Background art

A butterfly needle for medical use is composed of an elongate central body which acts as a needle hub, having two flexible wings bilaterally extending therefrom, which have the purpose of both allowing needle handling during injection into a blood vessel of a patient and fixing the needle to the epidermis of the patient by means of a plaster.

Typically, these butterfly needles also have safety devices which have the purpose of preventing operators from being accidentally injured after use thereof, during handling for disposal.

These safety devices consist of a spring having two elastically converging arms, which is housed in a container body associated with the proximal end of the butterfly needle.

The arms of the spring are held in a forced opened-apart state by the inner shape of the container body during use whereas, as the needle is retracted from the blood vessel of the patient, they become free to return to their natural, convergent position, thereby irreversibly overlapping the needle tip by their respective distal end portions, typically bent toward each other, and making it harmless.

In other words, once the needle has been used for an injection, the operator retracts it from the blood vessel thereby causing the spring to be automatically triggered, such that when the needle is entirely retracted its tip is entirely covered by the ends of the spring arms and remains harmless.

From US 6,749,588 a catheter and introducer needle assembly with needle shield are known.

The needle shield includes a means for preventing unwanted distal movement of the needle once the needle has been withdrawn into the needle shield. The needle shield also includes a means for connecting the needle shield to the catheter hub until the sharp distal tip of the introducer needle has been withdraw into the needle shield. Thus, when the distal end of the introducer needle extends form the distal portion of the needle shield, the needle shield is connected to the catheter hub and when the sharp distal end of the needle is withdrawn into the needle shield, the needle shield is disconnected from the catheter hub.

Form EP 2204204 a needle protection device is known.

The needle protection device of a generic medical instrument comprises a container provided with concentric through holes adapted to allow the needle to pass through them, a protection means movably housed inside the container where the protection means is provided with locking members adapted to cover the sharp end of the needle, locking the same inside the contained.

The above described prior art suffers from certain drawbacks.

A first drawback is that safety devices are housed in an additional container body, i.e. in a body that is specially designed for this purpose and is added to the structure of the butterfly needle, typically at its proximal end.

This arrangement causes inconvenient handling of butterfly needles with safety devices, also in view of the fact that healthcare operators wear protective gloves for making injections and have reduced sensitivity to touch and grip.

A second drawback is that prior art butterfly needles have flexible wings and, when the latter are grasped to make the injection, they may allow undesired deviations of the needle relative to the optimal direction to reach the blood vessel, due to the resistance opposed by organic tissues to penetration, which may force operators to repeat the injection, possibly multiple times, thereby causing discomfort and pain to the patient.

A further drawback is that a butterfly needle cannot be firmly grasped, due to the flat shape of its wings, which may slip through the fingers of healthcare operators, no arrangement being provided for guiding the proper finger position and a firm grasp.

Another drawback is that, when prior art needles are removed from the blood vessel of the patient, they carry blood residues in the needle shank, and such residues may uncontrollably drip, after full removal.

This is a particularly hazardous situation for operators that work in hospitals with patients having blood- and contact-transmissible infections, e.g. AIDS patients.

### Disclosure of the invention

The invention has the object to improve the prior art.

Another object of the invention is to provide a butterfly needle with a protective device that affords a considerable overall size reduction.

A further object of the invention is to provide a butterfly needle with a protective device that allows proper positioning of the fingers of a healthcare operator, as well as a firm and safe grasp.

Also, an object of the invention is to provide a butterfly needle with a protective device which, after use, allows any blood residues to be collected in a given location, such that healthcare operators are protected from any accidental contact hazards. The invention relates to a butterfly needle with a protective device, as defined by the features of claim 1.

The invention achieves the following advantages:
- improving grip and handleability of a butterfly needle;
- considerably reducing the overall size of a butterfly needle with a protective device; and
- after use, collecting any organic liquid (blood) residues in a specially-designed collection area, for accidental contact protection.

### Brief description of the drawings

Further features and advantages of the invention will be more readily apparent upon reading of the detailed description of preferred non-exclusive embodiments of a butterfly needle with a protective device, which is shown as a non-limiting example in the annexed drawings, in which:
FIG. 1 is a perspective view of a butterfly needle of the invention in a prior-to-use configuration;
FIG. 2 is a perspective view of the butterfly needle of Figure 1, in a ready-to-use configuration;
FIG. 3 is a perspective view of the butterfly needle of Figure 1, in a next-to-use configuration;
FIG. 4A is an exploded perspective view of the butterfly needle of Figure 1;
FIG. 4B is an exploded perspective view of the butterfly needle of Figure 4A in an inverted state;
FIG. 5 is a top view of the butterfly needle of the invention, with an interior viewing and in a ready-to-use configuration;
FIG. 6 is the corresponding side view of the butterfly needle of Fig. 5;
FIG. 7 is a top view of the butterfly needle of the invention, with an interior viewing and in a ready-to-use configuration during retraction thereof from a vessel of a patient and automatic actuation of a safety device;
FIG. 8 is the corresponding side view of the needle of Figure 7;
FIG. 9A is a perspective, enlarged view of a safety device equipping the butterfly needle of the invention in a ready-to-use configuration;
FIG. 9B is a perspective view as taken through a different angle as compared with Figure 9A and in a larger scale of a safety device equipping the butterfly needle of the invention in a ready-to-use configuration;
FIG. 10A is a perspective, enlarged view of the safety device of Figure 9A, in an actuated protective configuration;
FIG. 10B is a perspective, enlarged view of the safety device of Figure 9B, in an actuated protective configuration;
FIG. 11 is a perspective view of the proximal end of the butterfly needle of the invention, with the needle in a retracted configuration.

### Detailed description of a preferred embodiment

Referring to the above mentioned figures, numeral 1 generally designates a butterfly needle with a protective or safety device 2 adapted to be automatically triggered after use of the butterfly needle 1.

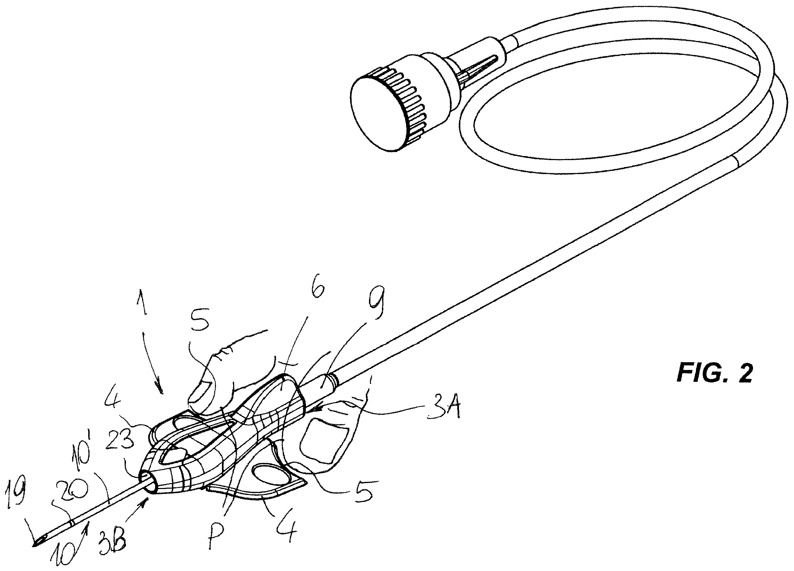

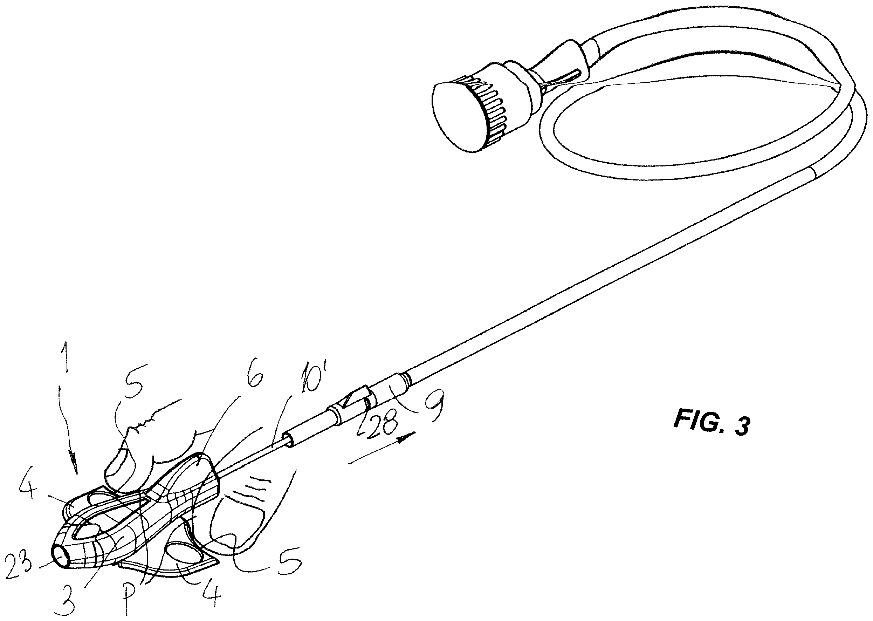

The butterfly needle comprises a central body 3 defining a proximal end 3A, i.e. facing an operator during use, and an opposite distal end 3B, with two rigid and substantially flat wings 4 bilaterally extending therefrom, which have a specially shaped profile with two concavities 5 on the edges facing the proximal end 3a, for receiving the fingertips "P" of an operator, as schematically shown in Figures 2 and 3.

As shown in the figures, the central body 3 forms a raised fin 6 at the proximal end 3A, which is designed to be grasped by the fingertips "P" for making the injection or retracting the butterfly needle 1.

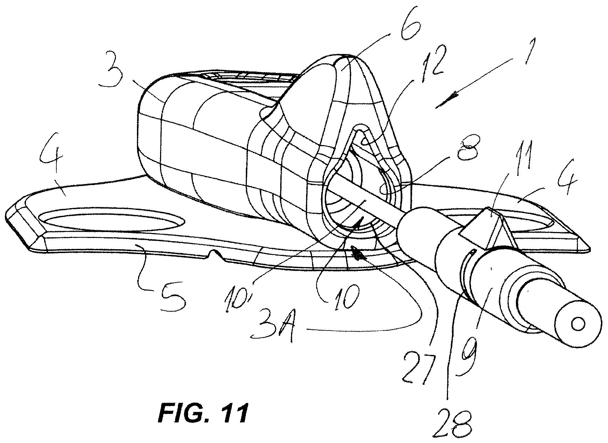

Referring to Figure 11, the central body 3 is illustrated with an aperture 8 at the proximal end 3A, whose contour is specially shaped for guided accommodation of a needle hub 9, which supports the shank 10' of the butterfly needle 1.

In order to prevent any undesired rotation between the central body 3 and the needle hub 9, the latter is equipped with a raised longitudinal rib 11 which is designed for mating engagement with a corresponding slot 12 formed in the aperture 8.

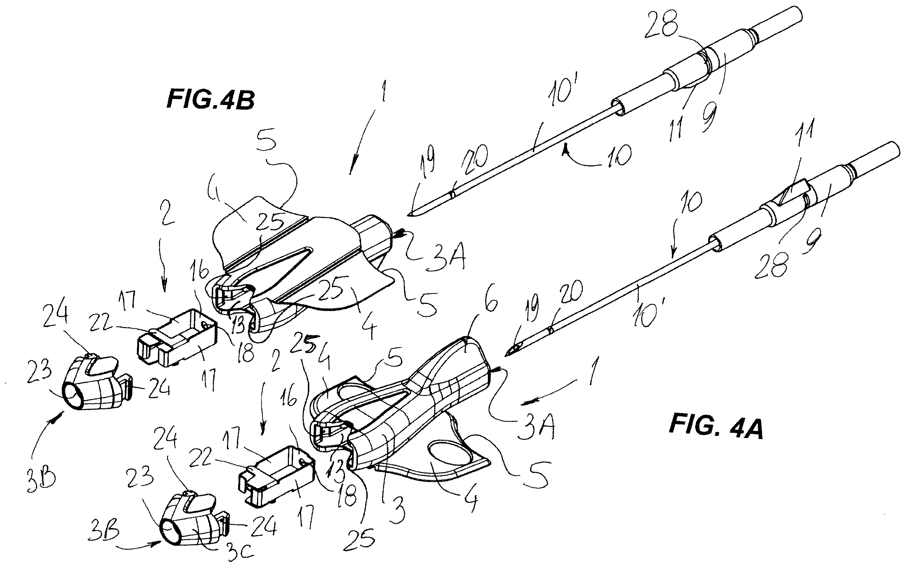

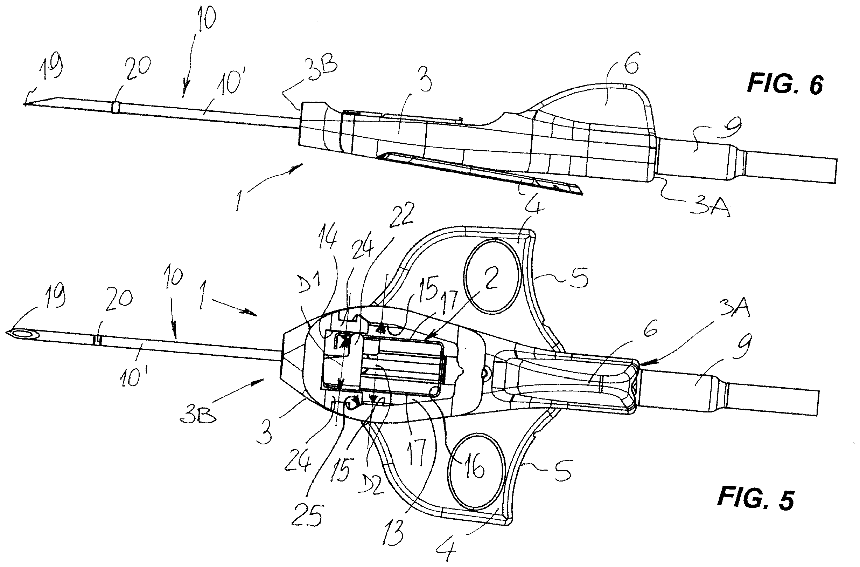

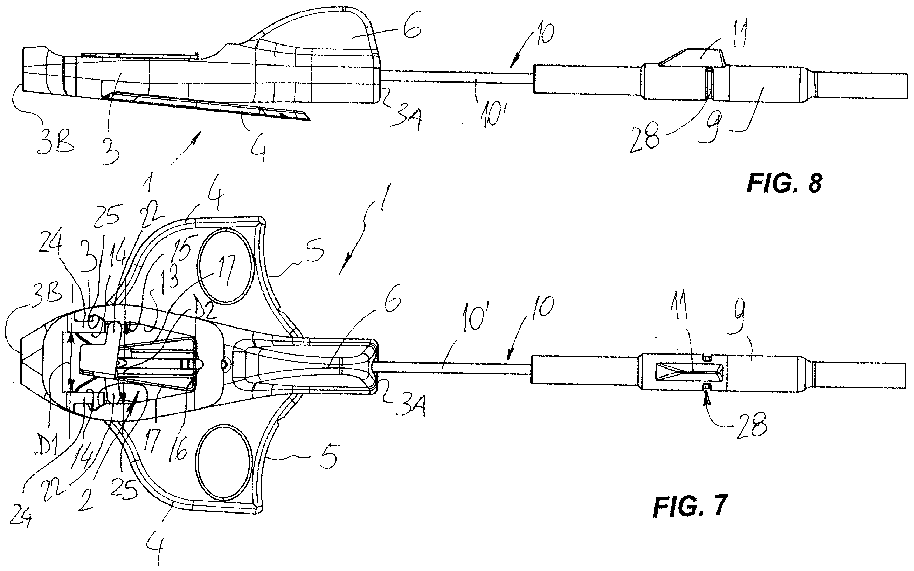

Referring now to Figures 4A, 5 and 7, it shall be noted that the central body 3 forms therein a housing chamber 13 for slidingly receiving the protective or safety device 2, such that the latter may be moved from a rest position (Figure 5) to an actuated position (Figure 7), as described in greater detail below.

The chamber 13 is divided into two zones, namely a first distal zone in which the first walls 14 thereof are at a first mutual distance "D1" and a second proximal zone in which the second walls 15 are at a second mutual distance "D2", which is greater than the first distance "D1".

As shown with reference to Figures 5, 7, 9A, 9B, 10A, 10B, the protective or safety device 2 consists in a specially shaped spring, which is composed of a bottom wall 16 oriented perpendicular to the shank 10' of the needle 10, and having two elastic arms 17 extending therefrom in the distal direction, which normally tend to converge toward each other.

The bottom wall 16 has a central hole 18 for the shank 10 of the needle 10 to extend therethrough and whose diameter is approximately a few tenths of a millimeter greater than the outside diameter of the shank 10' of the needle 10, which in turn has a bulge 20 at its tip 19, whose diameter is a few hundredths of a millimeter greater than the diameter of the hole 18, such that it cannot extend therethrough, whereby it engages against the bottom wall 16 when subjected to a retracting action.

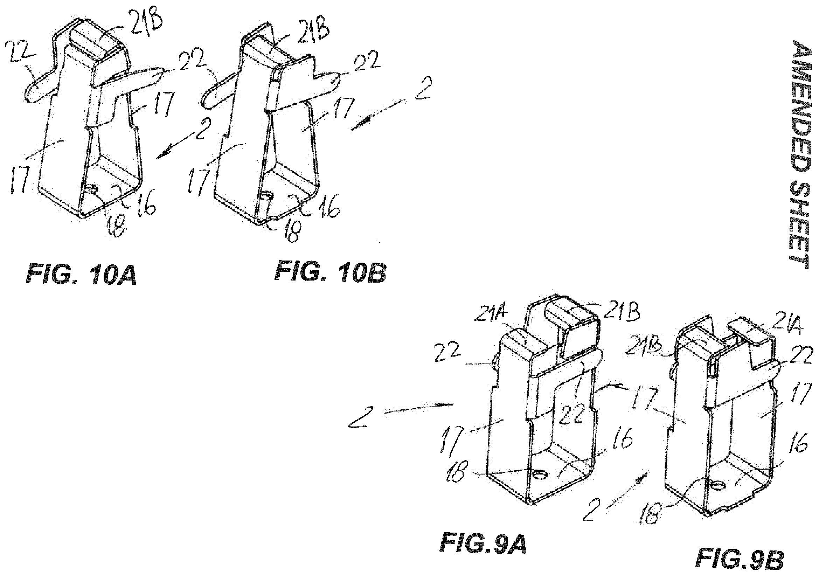

Each of the two arms 17 has its distal end folded to form a cantilever projection, 21 A and 21 B respectively, which projections face toward each other in planarly offset relationship, as shown in Figures 9A, 9B, 10A, 10B.

Furthermore, each of the arms 17 has a respective support foot 22 extending out of a lateral edge, perpendicular to its respective arm 17.

The feet 22 are designed to alternately rest on the walls 14 or 15 of the two zones of the chamber 13 according to the position of the protective device 2 in the chamber 13; when they rest on the walls 14 (Figure 5) the two arms 17 are forced to an opened-apart position, in which the protective or safety device 2 is idle, whereas when they rest on the walls 15, the two arms 17 are released into the mutually converging position (Figure 7) and the protective and safety device 2 is actuated.

Referring to Figures 2, 3, 4A, 4B it shall be noted that a receptacle 23 is formed in the distal end 3b of the central body 3 for collecting the organic liquid (blood) that keeps adhering to the shank 10' of the needle 10 as the latter is retracted from the blood vessel of the patient, and is rubbed off upon the mutual sliding motion of the shank 10' of the needle 10 and the central body 3.

As it may be expected, the bottom of the receptacle 23 has a hole (not shown) for the shank 10' of the needle 10 to snugly move and extend therethrough.

Referring to Figures 4A and 4B, it shall be noted that the central body 3 is typically composed of two mutually joined pieces, namely a ferrule 3C and the central body 3 itself.

The ferrule 3C is joined to the central body 3 by snap fit engagement, with two parallel tongues 24 forming respective pawls engaging in corresponding recesses 25 formed in the chamber 13, namely in the distal portion thereof.

Therefore, when the ferrule 3C is assembled, the two tongues 24 form the two first walls 14 of the chamber 13.

A snap fit engagement is provided between the proximal end 3A of the body 3, namely between the aperture 8 and the needle hub 9, which temporarily prevents any axial sliding movement between the body 3 and the needle hub 9, typically consisting of a raised transverse rib 27 extending from the inner wall of the aperture 8 and a corresponding perimeter groove 28 formed on the needle hub 9.

The operation of the butterfly needle with the protective device 2 is as follows: a healthcare operator grasps with his hands the fin 6 of the butterfly needle 1, by introducing his/her fingertips "P" into the two concavities 5 formed in the two rigid wings 4 for this purpose.

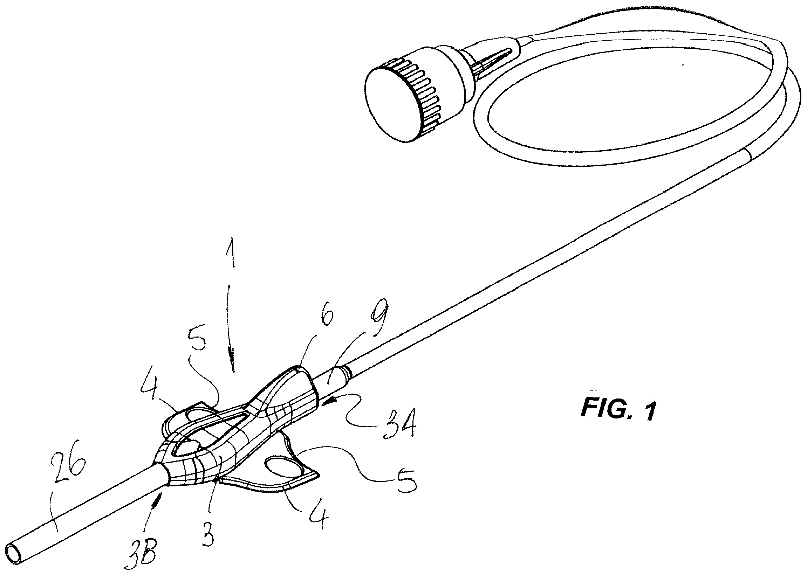

Once the central body 3 of the butterfly needle 1 has been firmly grasped, he/she removes the conventional protective shell 26 (Figure 1) of the shank 10' of the needle 10, whereupon the butterfly needle 1 is ready for injection into a blood vessel of a patient.

At this stage, the protective device 2 is in the idle position, i.e. it is situated in the distal zone of the chamber 13 with the arms 17 in an opened-apart position, because the support feet 22 rest on the first walls 14, as shown in Figure 5.

In this configuration, the longitudinal rib 11 is snugly received in the slot 12 of the aperture 8 and the needle hub 9 is neither able to rotate relative to the central body 3 nor to axially slide, due to the snap fit engagement of the rib 27 and the groove 28.

Once the healthcare operator has completed the injection, it removes the needle 10 with one hand, by pulling the needle hub 9 in the proximal direction, and overcoming the resistance of the snap fit engagement between the rib 27 and the groove 28, while temporarily holding the central body 3 on the epidermis of the patient with the other hand.

This retraction action in the proximal direction causes the bulge 20 to come to abutment against the bottom wall 16, without being able to pass through the hole 18 as the diameter of the latter is smaller than that of the bulge 20.

Due to this engagement the protective device 2 is moved in the proximal direction, whereby the support feet 22 slide from the first walls 14 to the second walls 15, while allowing the two arms 17 to converge toward each other and the two distal ends 21 a and 21 B to close over the tip 19 of the needle 10, thereby irreversibly covering it.

In this state, as shown in greater detail in Figure 7, the protective or safety device 2 is automatically actuated and the butterfly needle 1 becomes totally harmless.

The invention has been found to fulfill the intended objects.

The invention so conceived is susceptible to changes and variants within the inventive concept.

Also, all the details may be replaced by other technical equivalent elements.

In its practical implementation, any material, shape and size may be used as needed, without departure from the scope as defined by the following claims.

## Claims

1. A butterfly needle (1) with a protective device comprising:
- A central body (3) having one proximal end (3A) and one opposing distal end (3B);
- A couple of wings (4) that protrude bilaterally from said central body (3);
- A safety device (2) consisting in a specially shaped spring having a bottom wall (16) oriented perpendicular to the shank (10') of a needle (10), and having two elastic arms (17) extending therefrom in the distal direction which tend to converge toward each other, the bottom wall (16) having a central hole (18) for the shank (10') of the needle (10) which in turn has engaging means (20) larger than said hole (18) and engage said bottom wall (16) under traction;
- A needle (10) having a shank (10') provided with engaging means (20) located in the proximity to the tip (19) and which is supported by an hub (9) at an opposing proximal end, said needle (10) being slidingly received in said central body (3) longitudinally;
- said central body (3) comprising an inner housing chamber (13) located between said proximal end (3A) and said distal end (3B) and wherein said safety device (2) is irreversibly slidingly housed between a resting position and an actuated position, **characterized in that** said butterfly needle further comprises guiding (5, 6) means for its grasping and holding wherein said guiding means comprise a fin (6) raising from said proximal end (3A) of said central body (3) and a couple of concavities (5) obtained in said wings (4) in correspondence with said fin (6) and so shaped to receive the fingertips (P) of two fingers.

2. A butterfly needle as claimed in claim 1, wherein said central body (3) comprises collecting means (23) of residual organic fluids.

3. A butterfly needle as claimed in claim 2, wherein said collecting means comprise a collecting receptacle (23) obtained in said distal end (3B) of said central body (3) and slidingly passed through by said shank (10').

4. A butterfly needle as claimed in claim 1, wherein between said hub (9) and said central body (3), anti-rotational means (11, 12) of said shank (10') with respect of said central body (3) are provided for.

5. A butterfly needle as claimed in claim 4, wherein said anti-rotational means comprise:
- A longitudinal rib (11) which protrudes from said hub (9) and having an outer convex profile;
- A longitudinal cavity (12) obtained in said proximal end (3A) of said central body (3) and having an inner profile designed to be conjugate with said outer convex profile of said rib (11).

6. A butterfly needle as claimed in claim 1, wherein said inner housing chamber (13) comprises a first distal portion having a first width (D1) between first walls (14) and a second proximal portion prolonging from said first portion and having a second width (D2) between second walls (15), larger than said first width (D1).

7. A butterfly needle as claimed in claims 1 and 8, wherein said safety device (2) comprises a bottom wall (16) orthogonal to, and passed through by, said shank (10') from which two elastic arms (17) protrude, said arms (17) being normally converging elastically one toward the other and provided with resting means (22) alternatively on first walls (14) in a standing apart position or on second walls (15) in a reciprocally converging position.

8. A butterfly needle as claimed in claim 9, wherein said elastic arms (17) comprise distal end segments (21 A, 21 B) which are bound one toward the other in a misaligned position.

9. A butterfly needle as claimed in claim 7, wherein said elastic arms (17) comprise respective reciprocally faced resting feet (22) on said first walls (14) or second walls (15).

10. A butterfly needle as claimed in claim 1, wherein said wings are rigid wings (4).

## Patentansprüche

1. Schmetterlingsnadel (1) mit einer Schutzvorrichtung, umfassend:
- einen Zentralkörper (3) mit einem proximalen Ende (3A) und einem entgegengesetzten distalen Ende (3B);
- ein Flügelpaar (4), das beidseitig von dem Zentralkörper (3) herausragt;
- eine Sicherheitsvorrichtung (2), bestehend aus einer speziell geformten Feder mit einer Bodenwand (16) rechtwinklig zu dem Schaft (10') einer Nadel (10) orientiert, und mit zwei elastischen Armen (17), die sich in die distale Richtung davon erstrecken, welche dazu neigen zueinander zu konvergieren, wobei die Bodenwand (16) ein Zentralloch (18) für den Schaft (10') der Nadel (10) aufweist, der wiederum Eingreifmittel (20) größer als das Loch (18) aufweist und unter Zug mit der Bodenwand (16) in Eingriff sind;
- eine Nadel (10) mit einem Schaft (10'), der mit Eingreifmittel (20) ausgestattet ist, das sich in der Nähe der Spitze (19) befindet und das durch eine Nabe (9) am entgegengesetzten proximalen Ende gestützt wird, wobei die Nadel (10) in dem Zentralkörper (3) längs gleitend aufgenommen wird;
- der Zentralkörper (3) eine innere Gehäusekammer (13), angeordnet zwischen dem proximalen Ende (3A) und dem distalen Ende (3B), umfasst und wobei die Sicherheitsvorrichtung (2) irreversibel gleitend zwischen einer Ruheposition und einer betätigten Position beherbergt ist, **dadurch gekennzeichnet, dass** die Schmetterlingsnadel weiterhin Führungsmittel (5, 6) umfasst, um sie zu greifen und zu halten, wobei die Führungsmittel eine Flosse (6), die vom proximalen Ende (3A) des Zentralkörpers (3) zunimmt, und ein Paar Innenwölbungen (5), erhalten in den Flügeln (4) in Übereinstimmung mit der Flosse (6), und so gestaltet, um die Fingerspitzen (P) von zwei Fingern aufzunehmen, umfassen.

2. Schmetterlingsnadel nach Anspruch 1, wobei der Zentralkörper (3) Sammlungsmittel (23) von restlichen organischen Fluiden umfasst.

3. Schmetterlingsnadel nach Anspruch 2, wobei das Sammlungsmittel einen Sammlungsbehälter (23), erhalten in dem distalen Ende (3B) des Zentralkörpers (3) und gleitend durch den Schaft (10') geführt, umfasst.

4. Schmetterlingsnadel nach Anspruch 1, wobei zwischen der Nabe (9) und dem Zentralkörper (3) Antirotationsmittel (11, 12) des Schafts (10') hinsichtlich des Zentralkörpers (3) vorgesehen sind.

5. Schmetterlingsnadel nach Anspruch 4, wobei das Antirotationsmittel umfasst:
- eine Längsrippe (11), die aus der Nabe (9) herausragt und ein äußeres konvexes Profil aufweist;
- einen Längshohlraum (12), erhalten in dem proximalen Ende (3A) des Zentralkörpers (3) und mit einem inneren Profil, aufgebaut, um mit dem äußeren konvexen Profil der Rippe (11) übereinzustimmen.

6. Schmetterlingsnadel nach Anspruch 1, wobei die innere Gehäusekammer (13) umfasst einen ersten distalen Bereich mit einer ersten Breite (D1) zwischen ersten Wänden (14), und einen zweiten proximalen Bereich, der sich von dem ersten Bereich ausdehnt und mit einer zweiten Breite (D2) zwischen zweiten Wänden (15), größer als die erste Breite (D1).

7. Schmetterlingsnadel nach Ansprüchen 1 und 8, wobei die Sicherheitsvorrichtung (2) eine Bodenwand (16) orthogonal zu und geführt durch den Schaft (10'), von dem zwei elastische Arme (17) herausragen, umfasst, wobei die Arme (17) normal elastisch einer zu dem anderen konvergierend sind und ausgestattet mit Ruhemittel (22) abwechselnd an ersten Wänden (14) in einer stehenden Position voneinander oder an zweiten Wänden (15) in einer umgekehrt konvergierenden Position.

8. Schmetterlingsnadel nach Anspruch 9, wobei die elastischen Arme (17) distale Endsegmente (21A, 21B) umfassen, welche in einer verstellten Position aneinander gebunden sind.

9. Schmetterlingsnadel nach Anspruch 7, wobei die elastischen Arme (17) jeweilig umgekehrt zeigende Ruhe-Füße (22) an den ersten Wänden (14) oder zweiten Wänden (15) umfassen.

10. Schmetterlingsnadel nach Anspruch 1, wobei die Flügel starre Flügel (4) sind.

## Revendications

1. Aiguille à ailettes (1) munie d'un dispositif de protection (2), comprenant :
- un corps central (3) doté d'une extrémité proximale (3A) et d'une extrémité distale opposée (3B) ;
- deux ailettes (4) qui s'étendent de chaque côté dudit corps central (3) ;
- un dispositif de sécurité (2), constitué d'un ressort de forme particulière possédant une paroi de fond (16) orientée perpendiculaire à la tige (10') d'une aiguille (10), et possédant deux bras élastiques (17) s'étendant de celui-ci en direction distale, et venant converger l'un vers l'autre, la paroi de fond (16) comportant un trou central (18) pour la tige (10') de l'aiguille (10), possédant à son tour des moyens d'engagement (20) plus grands que ledit trou (18) et engageant ladite paroi de fond (16) sous traction ;
- une aiguille (10) comportant une tige (10') pourvue de moyens d'engagement (20) situés à proximité de la pointe (19), et supportée par une embase (9) à une extrémité proximale opposée, ladite aiguille (10) étant reçue longitudinalement coulissante dans ledit corps central (3) ;
- ledit corps central (3) comprenant une chambre de logement interne (13) entre ladite extrémité proximale (3A) et ladite extrémité distale (3B), et dans lequel ledit dispositif de sécurité (2) est logé coulissant de manière irréversible entre une position de repos et une position d'actionnement, **caractérisée en ce que** ladite aiguille à ailettes comprend par ailleurs des moyens de guidage (5, 6) permettant de la saisir et de la retenir, dans laquelle lesdits moyens de guidage comprennent une aile (6) s'élevant de ladite extrémité proximale (3A) dudit corps central (3), et une paire de creux (5) réalises dans lesdites ailettes (4) au niveau de ladite aile (6) et façonnés de manière à recevoir les bouts (P) de deux doigts.

2. Aiguille à ailettes selon la revendication 1, dans laquelle le corps central (3) comprend des moyens collecteurs (23) de fluides organiques résiduels.

3. Aiguille à ailettes selon la revendication 2, dans laquelle lesdits moyens collecteurs comprennent un réceptacle de collecte (23) réalisé dans ladite extrémité distale (3B) dudit corps central (3) et traversé de façon coulissante par ladite tige (10').

4. Aiguille à ailettes selon la revendication 1, dans laquelle des moyens anti-rotation (11, 12) sont agencés entre ladite embase (9) et ledit corps central (3), pour empêcher la rotation de ladite tige (10') par rapport audit corps central (3).

5. Aiguille à ailettes selon la revendication 4, dans laquelle lesdits moyens anti-rotation comprennent :
- une nervure longitudinale (11) faisant saillie de ladite embase (9) et possédant un profil extérieur convexe ;
- une cavité longitudinale (12) réalisée dans ladite extrémité proximale (3A) dudit corps central (3) et possédant un profil intérieur destiné à se conjuguer avec ledit profil extérieur convexe de ladite nervure (11).

6. Aiguille à ailettes selon la revendication 1, dans laquelle la dite chambre de logement interne (13) comprend une première partie distale ayant une première largeur (D1) entre des premières parois (14) et une seconde partie proximale se prolongeant de ladite première partie et ayant une seconde largeur (D2) entre des secondes parois (15), qui est supérieure à ladite première largeur (D1).

7. Aiguille à ailettes selon les revendications 1 et 8, dans laquelle ledit dispositif de sécurité (2) comprend une paroi de fond (16) orthogonale à est traversée par ladite tige (10') de laquelle font saillie deux bras élastiques (17), lesdits bras (17) convergeant normalement de façon élastique l'un vers l'autre et étant pourvus de moyens d'appui (22) pour s'appuyer alternativement sur les premières parois (14) dans une position d'écartement ou sur les secondes parois (15) dans une position de convergence réciproque.

8. Aiguille à ailettes selon la revendication 9, dans laquelle lesdits bras élastiques (17) comprennent des segments d'extrémité (21A, 21 B) liés l'un vers l'autre dans une position désalignée.

9. Aiguille à ailettes selon la revendication 7, dans laquelle lesdits bras élastiques (17) comprennent des pieds d'appui respectifs en regard l'un de l'autre sur lesdites premières parois (14) ou secondes parois (15).

10. Aiguille à ailettes selon la revendication 1, dans laquelle lesdites ailettes sont des ailettes rigides (4).
